# EUROPEAN PATENT APPLICATION

(11) **EP 3 889 651 A1**
(43) Date of publication of application: **06.10.2021**
(21) Application number: 19891966.4
(22) Date of filing: 01.04.2019
(51) Int. Cl.: G01T 1/202

(54) **PET DETECTOR HAVING SOME LIGHT GUIDES NOT CUT**

(30) Priority: 04.12.2018 CN 201811474956
(71) Applicant: Shandong Madic Technology Co., Ltd., Linyi, Shandong 276000 (CN)
(72) Inventor: LIU, Jiguo, Shandong 276000 (CN)
(74) Representative: Hoffmann Eitle
(86) International application number: PCT/CN2019/080781
(87) International publication number: WO 2020/113871

(57) **Abstract**

A PET detector having some light guides not cut, comprising a light guide bar array unit having some light guides not cut. The light guide bar array unit is in the form of an array consisting of a plurality of parallel light guide bars (2), and adjacent light guide bars (2) in some regions of the light guide bar array unit and a reflective material (4) between every two light guide bars are replaced with a light guide three-dimensional block having the identical shape and volume, taken as a whole. The detector sequentially comprises a layer formed by a scintillating crystal array unit, a layer formed by the light guide bar array unit, and a layer formed by a silicon photomultiplier array unit in an arrangement order.

## Description

### TECHNICAL FIELD

The present disclosure relates to the field of radiographic medical imaging equipment, and relates to a PET detector with partial non-cut light guides. The detector can be applied to medical imaging equipment including PET, SPECT and those with similar principles.

### BACKGROUND

In nuclear medicine imaging equipment such as PET and SPECT, it is required to measure the position and energy of rays. PET is the most typical medical imaging method that collects oppositely reflected photoelectrons (generally called true coincidence event LOR (line of reaction) and analyzes them accordingly to form an image. At present, the most common PET detector is a scintillator detector plus a photoelectric conversion device. After the rays hit scintillators, scintillation fluorescence is generated. The scintillation fluorescence is converted into an electrical signal by the photoelectric conversion device, and then sent to an electronic system for processing.

Commonly used photoelectric conversion devices include photomultiplier tubes, position-sensitive photomultiplier tubes and silicon photomultiplier tubes. The silicon photomultiplier tube is a semiconductor photoelectric conversion device that has become popular in recent years. The effect of the silicon photomultiplier tube is better than the photomultiplier tube and the position-sensitive photomultiplier tube, and the gain is close to that of the photomultiplier tube. It can be a single pixel at the time of purchase, or it can be an array of NxN (N ≥ 2) pixels, with the pixel size generally being 1mm-10mm. The silicon photomultiplier tube array is very similar in function to the position-sensitive photomultiplier tube, but it outputs signals in a way in which each pixel outputs a signal, which has a stronger recognition and positioning ability. The silicon photomultiplier tube array is similar in function to the position-sensitive photomultiplier tube, and the price is close to that of the photomultiplier tube. As a semiconductor device, there is room for further drop in the price of the silicon photomultiplier tube in case of mass production.

There are two ways to read out a signal of the photomultiplier tube. One is to directly digitize, and the other is to encode through the ANGER circuit (US3011057) and then digitize. The use of silicon photomultiplier tube is mainly the latter one, which facilitates taking advantage of its signal reception and transmission respectively. After being processed by the ANGER circuit, an output signal of a group of photomultiplier tubes can be encoded into three analog signals E, X and Y, so as to achieve the purpose of reducing the circuit scale.

There is such a solution in the prior art (CN201410648328.4), in which a layer of light guides is added between a crystal array and a silicon photomultiplier tube array, and the light is distributed with the aid of the light guides, with the purpose of being able to identify crystals smaller than the pixels of the silicon photomultiplier tubes and improve the resolution. However, the addition of the light guides in the prior art is only used for general auxiliary light distribution, and there is no further specific enlightenment or instruction.

There are also two ways to read out a signal of the silicon photomultiplier tube. One is to read out each pixel and then digitize it. Although this is accurate, the problem is that tens of thousands of signals are collected when only a single part is irradiated, which completely displays or even exceeds 4K high-definition. However, in most cases, such a high resolution is not required. The other way is to use an analog preprocessing (CN201410648328.4) circuit to preprocess a signal of the silicon photomultiplier tube array. Through the preprocessing, the signal of one array can be encoded into a minimum of 4 analog signals to achieve the purpose of reducing the circuit scale.

At present, the number of crystals in a common human body PET is more than 30,000, and can exceed 100,000 at the most, in which more than 30,000 silicon photomultiplier tube pixels are used. Generally, the commercially available silicon photomultiplier tube arrays are 2x2, 4x4 or 8x8, mainly 4x4. Taking 32,000 crystals as an example, it is assumed that approximately each crystal corresponds to one pixel, then the device needs 2,000 4x4 silicon photomultiplier tube arrays. Even if after encoding, the number of analog signal channels can still reach 8,000, and the circuit scale is very large.

At the same time, if the signal of each of the above scintillation crystal strips is collected by a light guide and then by a silicon photomultiplier tube correspondingly, all the occurrence times of PET can be collected objectively and truly, and obtained static or dynamic images of the PET will also reach the highest resolution level. However, in doing so, there are several problems with the current application conditions: 1) In many cases, such a high resolution is not needed. Even if there is a location of interest, it is only required to be able to accurately know about the region of the location of interest, and a too high resolution is not required; and 2) The excessive signal processing volume makes the circuit scale, amount of heat generation, and data processing volume large. The entire device is not only bulky, but also is prone to damage. Maintenance and cooling of the device both require a lot of capital, manpower and settings. In applications thereof, the loss often outweighs the gain. In the case of huge cost and complicated processing technique, the results obtained are not what are desired.

### SUMMARY

In view of the above problem in the prior art that each of the light guide strips is truthfully cut for collection, whereas such a large signal is not required in practice, the present disclosure provides a targeted solution, that is, part of the light guide strips is not cut or not completely cut; in this way, there is actually no loss when collecting and summarizing signals in pieces, but a lot of work of cutting the light guides is reduced, which is of great practical significance.

A light guide strip array unit with partial non-cut light guides is provided, in which: the light guide strip array unit is in the form of an array composed of a plurality of mutually parallel light guide strips, and the light guide strips are rectangular parallelepipeds having the same specifications in terms of each of the length, the width and the height; each two of the light guide strips are provided with a reflective material or plated with a reflective material therebetween; the adjacent light guide strips and the reflective materials between each two light guide strips in partial regions of the above light guide strip array unit are replaced with a monolithic light guide three-dimensional block having the same shape and volume as the partial regions, and the number of the partial regions is one or more.

Further, each of the partial regions is specifically an N×M rectangular region, where N and M are both positive integers and at least one of them is greater than 1, and N and M are equal or not equal to each other.

A PET detector with partial non-cut light guides is provided, in which: in the order of arrangement, the detector sequentially includes: a layer formed by scintillation crystal array units, a layer formed by the light guide strip array units as described above, and a layer formed by silicon photomultiplier tube array units; the layer formed by the scintillation crystal array units is formed by a plurality of scintillation crystal array units adjacent to each other, the layer formed by the light guide strip array units is formed by a plurality of light guide strip array units adjacent to each other, and the layer formed by the silicon photomultiplier tube array units is formed by a plurality of silicon photomultiplier tube array units adjacent to each other; the silicon photomultiplier tube array unit is an array aggregate formed by laterally arranging Q silicon photomultiplier tube arrays; the layer formed by the scintillation crystal array units is adjacent to the layer formed by the light guide strip array units; each independent light guide strip corresponds to the scintillation crystal strip with the same top-view projected area as the light guide strip, and each light guide three-dimensional block corresponds to several scintillation crystal strips with the same top-view projected area as the light guide block; the reflective material is selected from one or more of a super-grade retroreflective material, an engineering-grade reflective film, a high-grade reflective film, an enhanced spectrum reflective film, and a barium sulfate coating.

Further, P blocks in the silicon photomultiplier tube array unit are replaced with high-reflectivity material blocks, and the high-reflectivity material block is integrally formed by using a mold adapted to the shape of the silicon photomultiplier tube array unit, or is formed by cutting. The high-reflectivity material block is made entirely of a uniform high-reflectivity material, or a side of the high-reflectivity material block that faces the scintillation crystal array unit is coated with a high-reflectivity material, and the reflectivity of the side of the high-reflectivity material block that faces the scintillation crystal array unit is not less than 50%; Q and P are both natural numbers greater than 1, and M-N ≥ 1.

Further, part of regions of the silicon photomultiplier tubes of the silicon photomultiplier tube units that completely correspond to the top-view cross section of the light guide three-dimensional block is partially or completely replaced with the high-reflectivity material block.

A method for processing a PET detector signal is provided, which is aimed at the PET detector with partial non-cut light guides as described above and which includes: preprocessing, by an analog preprocessing circuit, all signals received by the silicon photomultiplier tube array unit, and encoding them into one or more analog signals; in which when all the signals received by the layer formed by the silicon photomultiplier tube array units are preprocessed by the analog preprocessing circuit, each signal transmitted through each light guide block is incorporated into a single signal.

Further, by incorporating each signal transmitted through each light guide block into a single signal, it means that the signals received by all the silicon photomultiplier tube arrays whose top-view cross-sectional area corresponds to or includes the area of each light guide block are incorporated into a single signal.

A method for processing a PET detector signal is provided, which is aimed at the PET detector with partial non-cut light guides as described above and which includes: preprocessing, by an analog preprocessing circuit, all signals received by the silicon photomultiplier tube array unit, and encoding them into one or more analog signals; in which when all the signals received by the layer formed by the silicon photomultiplier tube array units are preprocessed by the analog preprocessing circuit, each signal transmitted through each light guide block is incorporated into a single signal; by incorporating each signal transmitted through each light guide block into a single signal, it means that the signals received by all the silicon photomultiplier tube arrays whose top-view cross-sectional area corresponds to or includes the area of each light guide block are incorporated into a single signal; and before encoding, a preprocessed signal source of any of the encoded analog signals includes at least one silicon photomultiplier tube array.

The main idea of the present disclosure is explained as follows: in many cases, it is not necessary to collect the signals of each silicon photomultiplier tube in the form of one signal, but the signals of one array or even multiple arrays are collected, so that a certain resolution is sacrificed to greatly simplify the circuit and improve the efficiency. However, in this operation, it is also possible that the corresponding parts of the light guides are not cut, but a large block is retained. Due to the above simplification of the circuit, this operation greatly saves preparation time and effort and simplifies the process, but does not have a negative impact on the result. This point is not disclosed in the prior art and is non-obvious. In CN102787360A, it is introduced that reflective layers of different lengths are filled between multiple single composite crystals, and it is proposed to cut the crystals selectively; however, the purpose is to improve the resolution, and the process cost is increased. It completely contradicts to the present disclosure since the present disclosure aims to save the process, reduce process complexity and save manhours under the resolution that meets the requirements.

### BRIEF DESCRIPTION OF THE DRAWINGS

The accompanying drawings required to be used in the description of the embodiments of the present disclosure or the related art are described briefly below, so that the technical solutions according to the embodiments of the present disclosure or according to the related art will become clearer. It is apparent that the accompanying drawings in the following description show only some embodiments of the present disclosure. For those skilled in the art, other accompanying drawings may also be obtained according to these drawings provided, without any creative work.
FIG. 1 is a schematic view of a three-layer structure of the present disclosure when viewed from the side;
FIG. 2 is a schematic view showing 3*3 light guide strips are replaced with a light guide block;
FIG. 3 is a schematic view showing 4*4 light guide strips are replaced with a light guide block; and
FIG. 4 is a schematic view showing silicon photomultiplier tubes are partially replaced.

Parts corresponding to the reference signs: 1: scintillation crystal strip; 2: light guide strip; 3: silicon photomultiplier tube; 4: reflective material; 5: silicon photomultiplier tube array; 6: light guide block; 7: silicon photomultiplier tube array; 8: high-reflectivity block.

### DETAILED DESCRIPTION

Preferred embodiments of the present disclosure will be described in detail below with reference to the accompanying drawings, so that the advantages and features of the present disclosure can be more easily understood by those skilled in the art, thereby making a clearer and definite definition of the scope of protection of the present disclosure.

### First Embodiment

A light guide strip array unit with partial non-cut light guides is provided, in which: the light guide strip array unit is in the form of an array composed of a plurality of mutually parallel light guide strips, and the light guide strips are rectangular parallelepipeds having the same specifications in terms of each of the length, the width and the height; each two of the light guide strips are provided with a reflective material or plated with a reflective material therebetween; the adjacent light guide strips and the reflective materials between each two light guide strips in partial regions of the above light guide strip array unit are replaced with a monolithic light guide three-dimensional block having the same shape and volume as the partial regions, and the number of the partial regions is one or more.

Further, each of the partial regions is specifically an N×M rectangular region, N and M are both positive integers and at least one of them is greater than 1, and N and M are equal or not equal to each other.

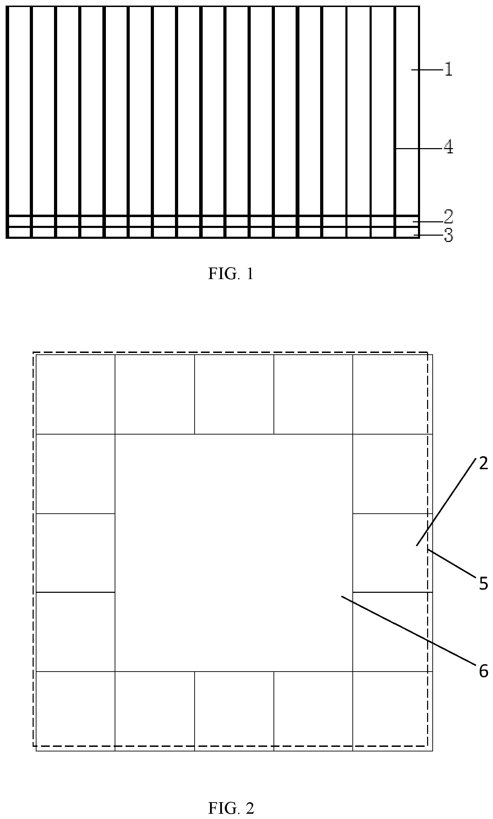

As shown in FIG. 2, N×M may be a light guide block having an area of 3*3 light guide strips, and the guided light signals are received by a 5*5 silicon photomultiplier tube array both before and after the replacement; under the premise that all the signals received by the array are guided into one signal, the replacement does not result in a reduction of the resolution, but the production workload is reduced.

### Second embodiment

A light guide strip array unit with partial non-cut light guides is provided, in which: the light guide strip array unit is in the form of an array composed of a plurality of mutually parallel light guide strips, and the light guide strips are rectangular parallelepipeds having the same specifications in terms of each of the length, the width and the height; each two of the light guide strips are provided with a reflective material or plated with a reflective material therebetween; the adjacent light guide strips and the reflective materials between each two light guide strips in partial regions of the above light guide strip array unit are replaced with a monolithic light guide three-dimensional block having the same shape and volume as the partial regions, and the number of the partial regions is one or more.

Further, each of the partial regions is specifically an N×M rectangular region, N and M are both positive integers and at least one of them is greater than 1, and N and M are equal or not equal to each other.

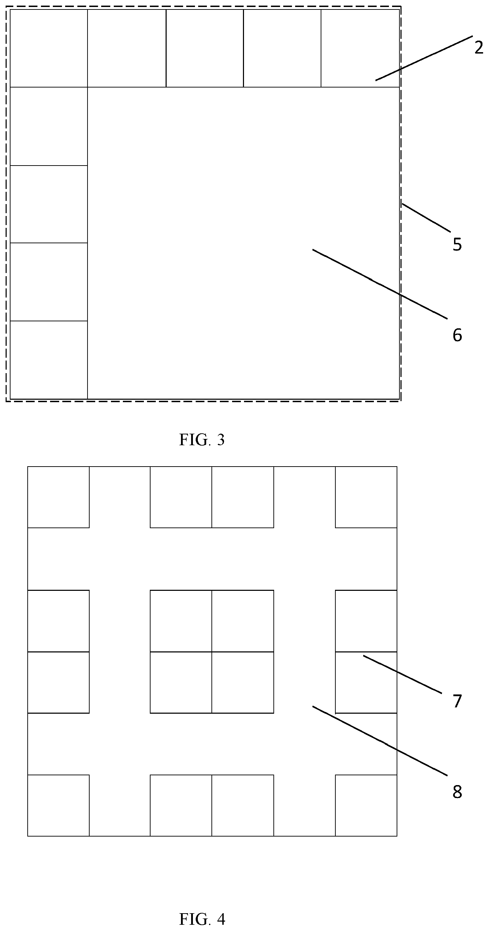

As shown in FIG. 3, N×M may be a light guide block having an area of 4*4 light guide strips, and the guided light signals are received by a 5*5 silicon photomultiplier tube array both before and after the replacement; under the premise that all the signals received by the array are guided into one signal, the replacement does not result in a reduction of the resolution, but the production workload is reduced.

However, in fact, if a monolithic design is adopted at the edge of the unit, the actual effect may be a little worse because of the possible diffusing phenomenon. It is more preferable that the light guide strips at the edge of the unit are not replaced.

### Third embodiment

A PET detector with partial non-cut light guides is provided, in which: in the order of arrangement, the detector sequentially includes: a layer formed by scintillation crystal array units, a layer formed by the light guide strip array units as described above, and a layer formed by silicon photomultiplier tube array units; the layer formed by the scintillation crystal array units is formed by a plurality of scintillation crystal array units adjacent to each other, the layer formed by the light guide strip array units is formed by a plurality of light guide strip array units adjacent to each other, and the layer formed by the silicon photomultiplier tube array units is formed by a plurality of silicon photomultiplier tube array units adjacent to each other; the silicon photomultiplier tube array unit is an array aggregate formed by laterally arranging Q silicon photomultiplier tube arrays; the layer formed by the scintillation crystal array units is adjacent to the layer formed by the light guide strip array units; each independent light guide strip corresponds to the scintillation crystal strip with the same top-view projected area as the light guide strip, and each light guide three-dimensional block corresponds to several scintillation crystal strips with the same top-view projected area as the light guide block; the reflective material is selected from one or more of a super-grade retroreflective material, an engineering-grade reflective film, a high-grade reflective film, an enhanced spectrum reflective film, and a barium sulfate coating. Herein, the signals transmitted by several crystal strips corresponding to the light guide block can only be incorporated into one signal during circuit processing, but this is completely acceptable when the resolution requirement is not high.

Further, P blocks in the silicon photomultiplier tube array unit are replaced with high-reflectivity material blocks, and the high-reflectivity material block is integrally formed by using a mold adapted to the shape of the silicon photomultiplier tube array unit, or is formed by cutting. The high-reflectivity material block is made entirely of a uniform high-reflectivity material, or a side of the high-reflectivity material block that faces the scintillation crystal array unit is coated with a high-reflectivity material, and the reflectivity of the side of the high-reflectivity material block that faces the scintillation crystal array unit is not less than 50%; Q and P are both natural numbers greater than 1, and M-N≥1. Further, part of regions of the silicon photomultiplier tubes of the silicon photomultiplier tube units that completely correspond to the top-view cross section of the light guide three-dimensional block is partially or completely replaced with the high-reflectivity material block.

The price of SiPMT here is also very expensive. When there is no need to construct a single circuit for 1*1 or a small area, most of the SiPMTs therein are replaced with high-reflectivity material blocks. Under the premise of saving costs, the signals can still be received. However, the imaging effect will not be affected in case of general resolution, such as 480P, 720P, etc.

### Fourth embodiment

In actual operation, when P blocks in the silicon photomultiplier tube array unit are replaced with high-reflectivity material blocks, a variety of configurations are possible. The isolated silicon photomultiplier tube array units may be at any distance from each other, as long as the signals can be received within a region. For the convenience of actual operation, the replacement may be specifically performed according to a certain ratio. For example, 5/9 or 1/2 of the silicon photomultiplier tubes are replaced, and the high-reflectivity blocks may be composed of several small pieces spliced together, or may be a whole piece. The vacant parts between the high-reflectivity blocks are filled with small silicon photomultiplier tubes or arrays. Taking FIG. 4 as an example, in fact, 5/9 of the SiMPT is replaced, and a pattern of regular replaced arrays is formed.

### Fifth embodiment

A method for processing a PET detector signal is provided, which is aimed at the PET detector with partial non-cut light guides as described above and which includes: preprocessing, by an analog preprocessing circuit, all signals received by the silicon photomultiplier tube array unit, and encoding them into one or more analog signals; in which when all the signals received by the layer formed by the silicon photomultiplier tube array units are preprocessed by the analog preprocessing circuit, each signal transmitted through each light guide block is incorporated into a single signal.

Further, by incorporating each signal transmitted through each light guide block into a single signal, it means that the signals received by all the silicon photomultiplier tube arrays whose top-view cross-sectional area corresponds to or includes the area of each light guide block are incorporated into a single signal.

### Sixth embodiment

A method for processing a PET detector signal is provided, which is aimed at the PET detector with partial non-cut light guides as described above and which includes: preprocessing, by an analog preprocessing circuit, all signals received by the silicon photomultiplier tube array unit, and encoding them into one or more analog signals; in which when all the signals received by the layer formed by the silicon photomultiplier tube array units are preprocessed by the analog preprocessing circuit, each signal transmitted through each light guide block is incorporated into a single signal; by incorporating each signal transmitted through each light guide block into a single signal, it means that the signals received by all the silicon photomultiplier tube arrays whose top-view cross-sectional area corresponds to or includes the area of each light guide block are incorporated into a single signal; before encoding, a preprocessed signal source of any of the encoded analog signals includes at least one silicon photomultiplier tube array.

Herein, under the premise that part or all of the regions of the silicon photomultiplier tubes is replaced with the high-reflectivity material blocks, the setting of the circuit signal here should also take into account that the corresponding SiMPT part of the encoding circuit includes at least SiMPT and the high-reflectivity material blocks. If there are only the high-reflectivity material blocks, it is obvious that no signals will be generated.

Described above are only specific embodiments of the present disclosure, but the scope of protection of the present disclosure is not limited to this. Any change or replacement that can be contemplated without creative work should be covered within the scope of protection of the present disclosure. Therefore, the scope of protection of the present disclosure shall be accorded with the scope of the claims.

## Claims

1. Alight guide strip array unit with partial non-cut light guides, wherein:
the light guide strip array unit is in the form of an array composed of a plurality of mutually parallel light guide strips, and the light guide strips are rectangular parallelepipeds having the same specifications in terms of each of the length, the width and the height; each two of the light guide strips are provided with a reflective material or plated with a reflective material therebetween;
the adjacent light guide strips and the reflective materials between each two light guide strips in partial regions of the light guide strip array unit are replaced with a monolithic light guide three-dimensional block having the same shape and volume as the partial regions; and
the number of the partial regions is one or more.

2. The light guide strip array unit with partial non-cut light guides according to claim 1, wherein:
each of the partial regions is specifically an N×M rectangular region, N and M are both positive integers and at least one of them is greater than 1, and N and M are equal or not equal to each other.

3. A PET detector with partial non-cut light guides, wherein:
in the order of arrangement, the detector sequentially comprises: a layer formed by scintillation crystal array units, a layer formed by the light guide strip array units according to claim 1 or 2, and a layer formed by silicon photomultiplier tube array units;
the layer formed by the scintillation crystal array units is formed by a plurality of scintillation crystal array units adjacent to each other, the layer formed by the light guide strip array units is formed by a plurality of light guide strip array units adjacent to each other, and the layer formed by the silicon photomultiplier tube array units is formed by a plurality of silicon photomultiplier tube array units adjacent to each other;
the silicon photomultiplier tube array unit is an array aggregate formed by laterally arranging Q silicon photomultiplier tube arrays;
the layer formed by the scintillation crystal array units is adjacent to the layer formed by the light guide strip array units; each independent light guide strip corresponds to the scintillation crystal strip with the same top-view projected area as the light guide strip, and each light guide three-dimensional block corresponds to several scintillation crystal strips with the same top-view projected area as the light guide block; and
the reflective material is selected from one or more of a super-grade retroreflective material, an engineering-grade reflective film, a high-grade reflective film, an enhanced spectrum reflective film, and a barium sulfate coating.

4. The PET detector with partial non-cut light guides according to claim 3, wherein:
P blocks in the silicon photomultiplier tube array unit are replaced with high-reflectivity material blocks, and the high-reflectivity material block is integrally formed by using a mold adapted to the shape of the silicon photomultiplier tube array unit, or is formed by cutting; the high-reflectivity material block is made entirely of a uniform high-reflectivity material, or a side of the high-reflectivity material block that faces the scintillation crystal array unit is coated with a high-reflectivity material, and the reflectivity of the side of the high-reflectivity material block that faces the scintillation crystal array unit is not less than 50%; and
Q and P are both natural numbers greater than 1, and M-N≥1.

5. The PET detector with partial non-cut light guides according to claim 4, wherein:
part of regions of the silicon photomultiplier tubes of the silicon photomultiplier tube units that completely correspond to the top-view cross section of the light guide three-dimensional block is partially or completely replaced with the high-reflectivity material block.

6. A method for processing a PET detector signal, which is aimed at the PET detector with partial non-cut light guides according to any one of claims 3 to 5, the method comprising:
preprocessing, by an analog preprocessing circuit, all signals received by the silicon photomultiplier tube array unit, and encoding them into one or more analog signals;
wherein when all the signals received by the layer formed by the silicon photomultiplier tube array units are preprocessed by the analog preprocessing circuit, each signal transmitted through each light guide block is incorporated into a single signal.

7. The method for processing the PET detector signal according to claim 6, wherein:
by incorporating each signal transmitted through each light guide block into a single signal, it means that the signals received by all the silicon photomultiplier tube arrays whose top-view cross-sectional area corresponds to or comprises the area of each light guide block are incorporated into a single signal.

8. A method for processing a PET detector signal, which is aimed at the PET detector with partial non-cut light guides according to any one of claims 4 to 5, the method comprising:
preprocessing, by an analog preprocessing circuit, all signals received by the silicon photomultiplier tube array unit, and encoding them into one or more analog signals;
wherein when all the signals received by the layer formed by the silicon photomultiplier tube array units are preprocessed by the analog preprocessing circuit, each signal transmitted through each light guide block is incorporated into a single signal;
by incorporating each signal transmitted through each light guide block into a single signal, it means that the signals received by all the silicon photomultiplier tube arrays whose top-view cross-sectional area corresponds to or comprises the area of each light guide block are incorporated into a single signal; and
before encoding, a preprocessed signal source of any of the encoded analog signals comprises at least one silicon photomultiplier tube array.
